(19) Europäisches Patentamt
European Patent Office
Office européen des brevets

(11) Veröffentlichungsnummer: **0 008 663**
**B1**

(12) # EUROPÄISCHE PATENTSCHRIFT

(45) Veröffentlichungstag der Patentschrift: **20.05.81**

(51) Int. Cl.³: **C 07 C 35/37**, C 07 F 7/02, A 61 K 7/46

(21) Anmeldenummer: **79102661.0**

(22) Anmeldetag: **26.07.79**

(54) **Methanonaphthaline, sowie ein Verfahren zu deren Herstellung und die Anwendung dieser Verbindungen zur Herstellung von Norpatchoulenol.**

(30) Priorität **10.08.78 CH 8520/78**

(43) Veröffentlichungstag der Anmeldung: **19.03.80 Patentblatt 80/6**

(45) Bekanntmachung des Hinweises auf die Patenterteilung: **20.05.81 Patentblatt 81/20**

(84) Benannte Vertragsstaaten: **CH DE FR GB NL**

(56) Entgegenhaltungen:
**DE-A1-2 529 603**
**DE-A1-2 537 417**
**DE-A-1-2 739 449**
**DE-A-2 163 394**
**DE-A-2 407 782**
**CH-A-577 947**
**US-A-3 925 486**
**US-A-3 989 760**
**US-A-3 996 169**

(73) Patentinhaber: **L. Givaudan & Cie Société Anonyme, Patentdienst Postfach, CH-4002 Basel (CH)**

(72) Erfinder: **Oppolzer, Wolfgang, Prof. Dr., 4b Chemin de la Cocuaz, CH-1253 Vandoeuvres (CH)**

(74) Vertreter: **Aschert, Hubert et al, Postfach 601 Grenzacherstrasse 124, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

EP 0 008 663 B1

BUNDESDRUCKEREI BERLIN

Methanonaphthaline, sowie ein Verfahren zu deren Herstellung und die Anwendung dieser Verbindungen zur Herstellung von Norpatchoulenol

Die vorliegende Erfindung betrifft Methanonaphthaline, sowie ein Verfahren zu deren Herstellung und die Anwendung dieser Verbindungen zur Herstellung von Norpatchoulenol.

Die Methanonaphthaline der Erfindung haben die Formel

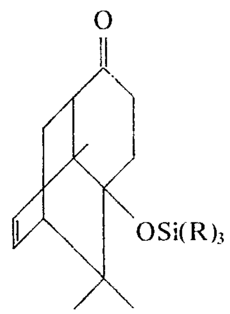

(V)

worin R eine niedere Alkylgruppe, gegebenenfalls nieder Alkyl-substituiertes Phenyl oder Phenylniederalkyl bedeutet, worin die niederen Alkylgruppen 1–4 Kohlenstoffatome enthalten.

Diese Verbindungen sind wertvolle Zwischenprodukte für die Herstellung von Norpatchoulenol.

Die Substituenten R können gleich oder voneinander verschieden sein und beispielsweise Methyl, Äthyl, Propyl, Butyl, Phenyl, Benzyl oder Tolyl darstellen. Vorzugsweise sind die Substituenten R jeweils gleich und bedeuten Methyl oder Äthyl.

Norpatchoulenol mit der Formel

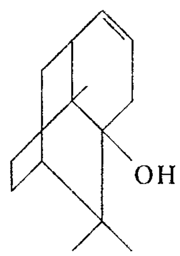

(VIII)

ist eine Komponente des Patchouliöls, in welchem es in sehr geringen Mengen vorkommt, dabei aber doch den Hauptgeruchsvektor von Patchouliöl darstellt. Norpatchoulenol kann für die Herstellung von Parfums und Riechstoffkompositionen verwendet werden, wobei in vielen Fällen das Norpatchoulenol für diesen Zweck besser geeignet ist, als Patchouliöl selbst. Es ist daher wünschenswert, Norpatchoulenol in reinem Zustand, frei von anderen im Patchouliöl vorhandenen Ingredientien, von welchen einige stören, zu erhalten. Die erste Synthese von Norpatchoulenol wurde in »Recherches«, (Roure Bertrand Dupont) 19 (1974) 69 beschrieben. Diese Synthese ist aber verhältnismäßig umständlich und beinhaltet eine Vielzahl von Stufen. Ein weiterer Weg zum Norpatchoulenol geht vom Patchoulialkohol aus, welcher in viel größerer Menge (etwa 40%) in Patchouliöl enthalten ist. Der Patchoulialkohol kann durch mikrobiologische Hydroxylierung (DOS 2 739 449) und darauf folgende oxydative Decarboxylierung (DOS 2 529 603) in Norpatchoulenol übergeführt werden. Diese Synthese ist jedoch nach wie vor vom aus der Natur gewonnenen Patchouliöl abhängig.

Die vorliegende Erfindung betrifft auch die Anwendung der Methanonaphthaline der Formel V zur Herstellung von Norpatchoulenol. Damit ist es möglich, Norpatchoulenol aus leicht zugänglichen Ausgangsmaterialien in einfacher Weise, guter Qualität und mit guter Gesamtausbeute herzustellen.

Die vorliegende Erfindung betrifft auch ein Verfahren zur Herstellung von Methanonaphthaline der Formel V, welches dadurch gekennzeichnet ist, daß man eine Verbindung der Formel

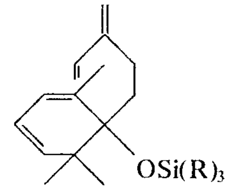

(IV)

worin R eine niedere Alkylgruppe, gegebenenfalls nieder Alkyl-substituiertes Phenyl oder Phenylniederalkyl bedeutet, worin die niederen Alkylgruppen 1–4 Kohlenstoffatome enthalten,

intramolekular zu einer Verbindung der Formel

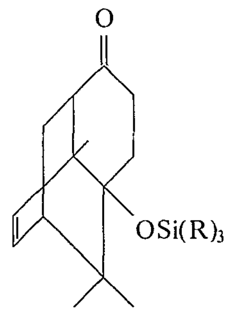

(V)

cyclisiert.

Die intramolekulare Cyclisierung kann in der Gasphase oder in flüssiger Phase durchgeführt werden. Bei Durchführung der Cyclisierung in flüssiger Phase, wird als Lösungsmittel zweckmäßig ein organisches Lösungsmittel, beispielsweise ein aromatischer oder aliphatischer Kohlenwasserstoff oder ein chlorierter Kohlenwasserstoff verwendet, z. B. Benzol, Toluol, Heptan, Decan oder Chlorbenzol.

In der flüssigen Phase wird die intramolekulare Cyclisierung zweckmäßig bei Temperaturen zwischen etwa 200 und etwa 300° C durchgeführt, wobei der bevorzugte Temperaturbereich zwischen 220 und 250° C liegt. Bei höheren Temperaturen werden im allgemeinen die Ausbeuten niedriger. Bei einer Temperatur von etwa 230° C beträgt die Reaktionsdauer etwa 12 Stunden.

Die intramolekulare Cyclisierung in der Gasphase kann bei Temperaturen im Bereich zwischen etwa 500 und etwa 800° C durchgeführt werden. Hierbei beträgt die Reaktionszeit im allgemeinen weniger als 2 Sekunden, vorzugsweise etwa 0,5 Sekunden. Die Reaktion in der Gasphase wird zweckmäßig in einem Quarzrohr durchgeführt.

Die vorliegende Erfindung betrifft auch die Anwendung von Methanonaphthalinen der Formel V worin R eine niedere Alkylgruppe, gegebenenfalls nieder Alkyl-substituiertes Phenyl oder Phenyl-niederalkyl bedeutet, worin die niederen Alkylgruppen 1—4 Kohlenstoffatome enthalten, zur Herstellung von Norpatchoulenol der Formel

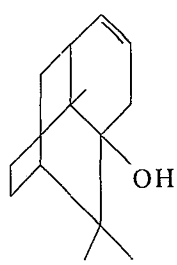

(VIII)

dadurch gekennzeichnet, daß man in beliebiger Reihenfolge die trisubstituierte Silylgruppe abspaltet, die 7,8-Doppelbindung zu einer Einfachbindung hydriert, ein Hydrazonderivat der so gebildeten 4-Ketoverbindung bildet und die Hydrazongruppe unter Bildung einer 3,4-Doppelbindung abbaut, wobei man Norpatchoulen erhält.

Die verschiedenen Stufen, in welchen die Verbindung der Formel V in das Norpatchoulenol übergeführt werden kann, können zweckmäßig in der folgenden Reihenfolge durchgeführt werden: (a) Abspaltung der trisubstituierten Silylgruppe, (b) Hydrierung der 7,8-Doppelbindung und (c) Entfernung der 4-Ketofunktion unter Einführung der 3,4-Doppelbindung. Diese Stufen können jedoch auch in anderer Reihenfolge durchgeführt werden.

Die Abspaltung der trisubstituierten Silylgruppe wird zweckmäßig unter den Bedingungen einer milden sauren Hydrolyse, beispielsweise mittels einer schwachen organischen Säure, wie verdünnter Essigsäure durchgeführt, wobei man zweckmäßig in Lösung in einem organischen Lösungsmittel arbeitet.

Die 7,8-Doppelbindung wird zweckmäßig unter Verwendung eines Edelmetallkatalysators hydriert. Als solchen Katalysator kann beispielsweise ein Platinkatalysator oder ein Palladiumkatalysator, z. B. Platinoxyd verwendet werden.

Die Entfernung der 4-Ketogruppe und die Einführung der 3,4-Doppelbindung kann zweckmäßig in an sich bekannter Weise unter Verwendung der Bamford-Stevens-Reaktion durchgeführt werden, d. h., daß man zuerst ein Hydrazonderivat bildet, beispielsweise ein p-Toluolsulfonylhydrazonderivat, welches dann abgespalten wird (Org. Reactions 23 (1976) 405).

Das Ausgangsmaterial der Formel IV kann in an sich bekannter Weise hergestellt werden, beispielsweise durch Behandlung eines 2,2,6-Trimethylcyclohexa-2,4-dienons mit einem 3-trisubstituierten Silyloxydienyl-lithium der Formel

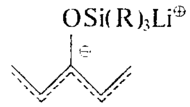

(II)

worin R die obige Bedeutung hat, und die gestrichelten Linien die Gegenwart von zwei konjugierten Doppelbindungen andeuten,

und darauffolgende Behandlung des erhaltenen Produktes mit einem trisubstituierten Silylhalogenid, vorzugsweise einem Triniederalkylsilylhalogenid, beispielsweise Trimethylsilylchlorid, unter Bildung des Tetraens der Formel

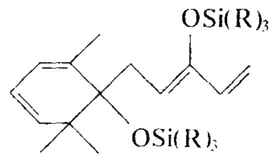

(III)

Die selektive Desiylierung dieses Tetraens mit Kaliumfluorid in Methanol bei einer Temperatur zwischen etwa 0 und 5° C ergibt nach Chromatographie das gewünschte Ausgangsmaterial der Formel

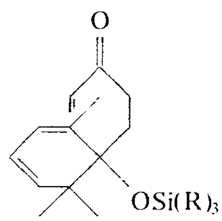

(IV)

Der Gesamtverlauf der Synthese von Norpatchoulenol, ausgehend von 2,2,6-Trimethylcyclohexa-2,4-dienon, kann durch das folgende Formelschema wiedergegeben werden.

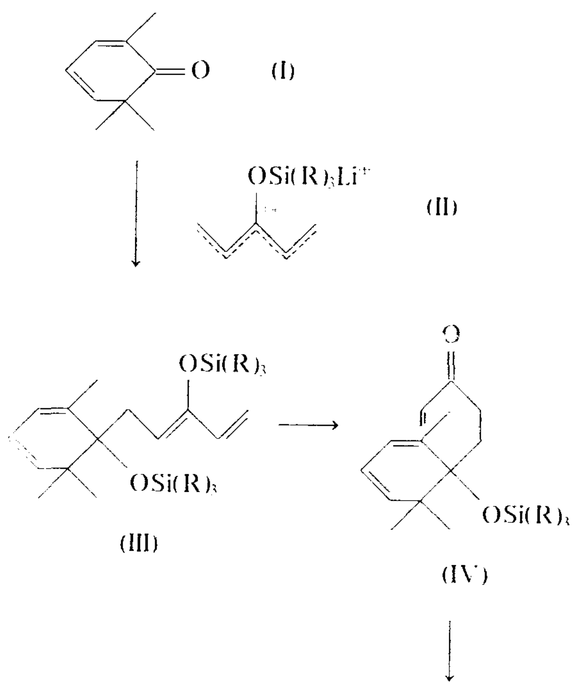

(I)

(II)

(III)

(IV)

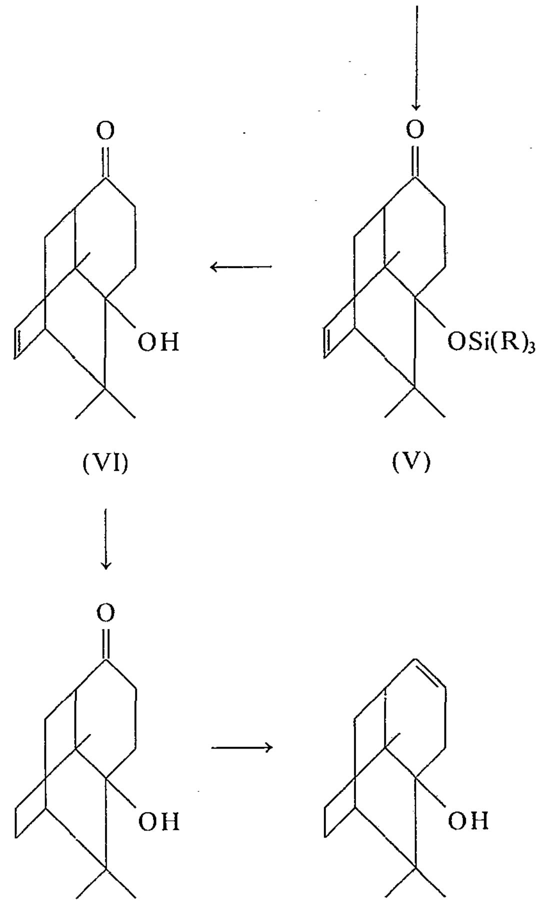

(VI) (V)

(VII) (VIII)

Das erfindungsgemäße Verfahren ermöglicht es sowohl (±), als auch (+), als auch (−)-Norpatchoulenol herzustellen, und zwar je nach der Konfiguration des verwendeten Ausgangsmaterials der Formel IV. Bei Verwendung eines optisch aktiven Ausgangsmaterials erhält man dementsprechend ein optisch aktives Endprodukt. Zur Herstellung des (+)-Norpatchoulenols, wie es in natürlichem Patchouliöl vorkommt, ist eine trisubstituierte Silylverbindung der Formel IV zu verwenden, welche in S-Konfiguration vorliegt.

## Beispiel 1

Eine Lösung von 20 mg 1-[3-Oxo-pent-4-en-1-yl]-1-trimethylsilyloxy-2,2,6-trimethylcyclohexa-2,4-dien in 6,6 ml absolutem Benzol wurde in einer silylierten (d. h. mit Bissilylacetamid behandelten) Pyrex-Ampulle eingeschmolzen, 12 Stunden auf 230° C erhitzt und anschließend über Silicagel (mit $CH_2Cl_2$) filtriert. Das eingedampfte Eluat ergab 15,2 g 1,2,3,4,4a,5,6,8a-Octahydro-2-oxo-4a-trimethylsilyloxy-5,5,8a-trimethyl-1,6-methanonaphthalin (76% Ausbeute, Smp. 73−76° C).

Das Ausgangsmaterial kann wie folgt hergestellt werden:

(a) Zu einer gerührten Lösung von 272 mg 2,2,6-Trimethylcyclohexa-2,4-dienon in 2 ml Tetrahydrofuran wurde eine Lösung von 2 mMol frisch bereitetem 3-Triäthylsilyloxypentadienyl-lithium in 4 ml Tetrahydrofuran bei −78° C zugetropft. Das Reaktionsgemisch wurde 5 Minuten bei −78° C gerührt, dann mit 1 ml Hexamethylphosphorsäuretriamid, darauffolgend mit 0,4 ml Trimethylsilylchlorid (0,4 ml) versetzt, eine weitere Stunde bei −78° C gerührt und schließlich auf gesättigte wäßrige $NH_4Cl$-Lösung gegossen. Extraktion mit Pentan und Eindampfen der getrockneten Extrakte ergab ein farbloses Öl, das in 30 ml Methanol gelöst wurde. Zu dieser Lösung wurden bei 0° C unter Argon und Rühren portionenweise 400 mg Kaliumfluorid zugesetzt. Das Gemisch wurde 1 Stunde bei 0 bis 5° C gerührt, anschließend auf gesättigte wäßrige $NH_4Cl$-Lösung gegossen und mit Äther extrahiert. Chromatographie der eingedampften Extrakte (Kieselgel, 15 g/$CH_2Cl_2$) ergab 276 mg 1-[3-Oxo-pent-4-en-1-yl]-1-trimethylsilyloxy-2,2,6-trimethylcyclohexa-2,4-dien (47% Ausbeute), farbloses Öl, IR($CCl_4$): 1710, 1693, 1625, 1258, 1130, 905 $cm^1$).

(b) Zu einer Lösung von 400 mg 3-Trimethylsilyloxy-1,4-pentadien in 3 ml Tetrahydrofuran unter Argon wurde eine Lösung von 2,3 ml sec-Butyllithium in Cyclohexan und nachher eine Lösung von 275 mg 2,6,6-Trimethylcyclohexa-2,4-dienon in 1 ml Tetrahydrofuran zugegeben. Nach 5 Minuten wurde 332 mg Trimethylsilylchlorid in 2 ml Hexamethylphosphorsäuretriamid zugesetzt und das Gemisch eine Stunde bei −78° gelassen. Nach Nacharbeitung [Siehe Beispiel 1 (a)] wurde das rohe Produkt mit 20 ml Methanol verdünnt bis 0° gekühlt und 224 mg Kaliumfluorid wurde zugegeben. Das Gemisch wurde 1 Stunde bei 0 bis 5°C gerührt, anschließend auf gesättigte wäßrige $NH_4Cl$-Lösung gegossen und mit Äther extrahiert. Chromatographie der eingedampften Extrakte ($SiO_2$, Toluen) ergab 329 mg 1-[3-Oxo-pent-4-en-1-yl]-trimethylsilyloxy-2,2,6-trimethylcyclohexa-2,4-dien (56% Ausbeute), charakterisiert wie oben.

Beispiel 2

Eine Lösung von 21 mg 1,2,3,4,4a,5,6,8a-Octahydro-2-oxo-4a-trimethylsilyloxy-5,5,8a-trimethyl-1,6-methanonaphthalin in 4 ml Essigsäure/Tetrahydrofuran/Wasser (3 : 1 : 1) wurde unter Argon bei 25°C über 10 Stunden gerührt, dann auf gesättigte wäßrige $NaHCO_3$-Lösung gegossen und mit $CH_2Cl_2$ extrahiert. Nach Chromatographie ($SiO_2$, Essigester/$CH_2Cl_2$) und Sublimation erhielt man 15 mg 1,2,3,4,4a,5,6,8a-Octahydro-2-oxo-4a-hydroxy-5,5,8a-trimethyl-1,6-methanonaphthalin (Smp. 112–115°C, 95% Ausbeute).

Beispiel 3

22 mg 1,2,3,4,4a,5,6,8a-Octahydro-2-oxo-4a-hydroxy-5,5,8a-trimethyl-1,6-methanonaphthalin in 4 ml Äthanol wird in Gegenwart von 6 mg $PtO_2$ 12 Stunden unter $H_2$-Atmosphäre (1 atm, 25°C) gerührt. Das durch Celit filtrierte und eingeoampfte Reaktionsgemisch ergab nach Chromatographie und Sublimation 20,5 mg Perhydro-2-oxo-4a-hydroxy-5,5,8a-trimethyl-1,6-methanonaphthalin (Smp. 120–123°C, 92% Ausbeute).

Beispiel 4

9,3 mg N-Toluolsulfonylhydrazid wurde zu einer Lösung von 11,1 mg 1,2,3,4,4a,5,6,7,8,8a-Decahydro-2-oxo-4a-hydroxy-5,5,8a-trimethyl-1,6-methanonaphthalin in heißer Essigsäure zugegeben. Das Gemisch wurde 10 Minuten unter Rückfluß zum Sieden erhitzt, anschließend auf gesättigte wäßrige $NaHCO_3$-Lösung gegossen, mit $CH_2Cl_2$ extrahiert und eingedampft. Die Lösung des Rückstandes in 4 ml Äther wurde langsam mit einer 1 N Lösung von Methyllithium in Äther (0,225 ml) bei 25°C versetzt, dann 12 Stunden unter Argon bei 25°C gerührt, dann auf Wasser gegossen und mit $CH_2Cl_2$ extrahiert. Chromatographie ($SiO_2$/$CH_2Cl_2$) und Sublimation bei 100–120°C ergab (±)-Norpatchoulenol (Smp. 135–141°C, 6,8 mg, 66% Ausbeute).

**Patentansprüche**

1. Methanonaphthaline der Formel

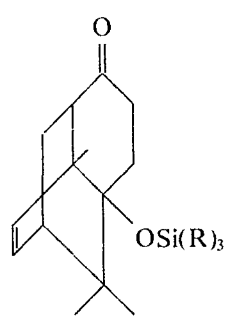

(V)

worin R eine niedere Alkylgruppe, gegebenenfalls nieder Alkyl-substituiertes Phenyl oder Phenylniederalkyl bedeutet, worin die niederen Alkylgruppen 1–4 Kohlenstoffatome enthalten.

2. Methanonaphthaline nach Anspruch 1, dadurch gekennzeichnet, daß R Methyl oder Äthyl bedeutet.

3. Verfahren zur Herstellung von Methanonaphthalinen der Formel

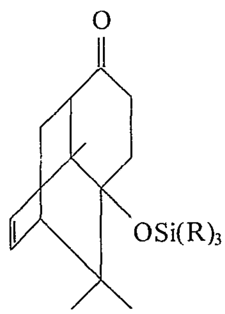

(V)

dadurch gekennzeichnet, daß man eine Verbindung der Formel

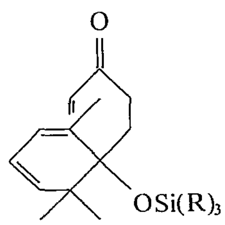

(IV)

worin R eine niedere Alkylgruppe, gegebenenfalls nieder Alkyl-substituiertes Phenyl oder Phenylniederalkyl bedeutet, worin die niederen Alkylgruppen 1—4 Kohlenstoffatome enthalten, intramolekular zu einer Verbindung der Formel V cyclisiert.

4. Verfahren nach Anspruch 3, dadurch gekennzeichnet, daß man ein Ausgangsmaterial verwendet, worin R eine Methyl- oder Äthylgruppe bedeutet.

5. Verfahren nach Anspruch 3 oder 4, dadurch gekennzeichnet, daß man die Cyclisierung in flüssiger Phase durchführt.

6. Verfahren nach Anspruch 5, dadurch gekennzeichnet, daß man die Cyclisierung in einem inerten organischen Lösungsmittel ausführt.

7. Verfahren nach Anspruch 6, dadurch gekennzeichnet, daß man als organisches Lösungsmittel einen aromatischen oder aliphatischen Kohlenwasserstoff verwendet.

8. Verfahren nach einem der Ansprüche 3—7, dadurch gekennzeichnet, daß man die Cyclisierung bei einer Temperatur zwischen 200 und 300° C durchführt.

9. Verfahren nach Anspruch 8, dadurch gekennzeichnet, daß man die Cyclisierung bei einer Temperatur zwischen 220 und 250° C durchführt.

10. Verfahren nach Anspruch 3 oder 4, dadurch gekennzeichnet, daß man die Cyclisierung in der Gasphase durchführt.

11. Verfahren nach Anspruch 10, dadurch gekennzeichnet, daß man die Cyclisierung bei einer Temperatur zwischen 500 und 800° C durchführt.

12. Anwendung von Methanonaphthalinen der Formel

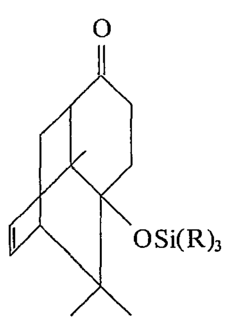

(V)

worin R eine niedere Alkylgruppe, gegebenenfalls nieder Alkyl-substituiertes Phenyl oder Phenylniederalkyl bedeutet, worin die niederen Alkylgruppen 1—4 Kohlenstoffatome enthalten,

zur Herstellung von Norpatchoulenol der Formel

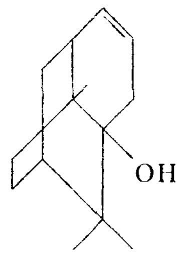

(VIII)

dadurch gekennzeichnet, daß man in beliebiger Reihenfolge die trisubstituierte Silylgruppe abspaltet, die 7,8-Doppelbindung zu einer Einfachbindung hydriert, ein Hydrazonderivat der so gebildeten 4-Ketoverbindung bildet und die Hydrazongruppe unter Bildung einer 3,4-Doppelbindung abbaut, wobei man Norpatchoulenol erhält.

13. Anwendung nach Anspruch 12, dadurch gekennzeichnet, daß man die trisubstituierte Silylgruppe durch milde saure Hydrolyse abspaltet.

14. Anwendung nach Anspruch 12 oder 13, dadurch gekennzeichnet, daß man die Hydrierung der 7,8-Doppelbindung unter Verwendung eines Edelmetallkatalysators durchführt.

15. Anwendung nach Anspruch 14, dadurch gekennzeichnet, daß man als Edelmetallkatalysator einen Platin- oder Palladium-Katalysator verwendet.

16. Anwendung nach einem der Ansprüche 12—15, dadurch gekennzeichnet, daß man als Hydrazonderivat ein p-Toluolsulfonylhydrazon bildet.

17. Anwendung nach einem der Ansprüche 12—16, dadurch gekennzeichnet, daß man das Ausgangsmaterial der Formel IV in S-Konfiguration verwendet, wobei man als Endprodukt der Formel VIII (+)-Norpatchoulenol erhält.

**Claims**

1. Methanonaphthalenes of the formula

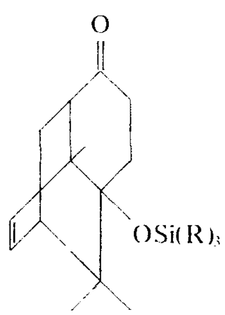

(V)

wherein R signifies a lower alkyl group, phenyl optionally lower alkyl-substituted or phenyl-lower alkyl, wherein the lower alkyl groups contain 1—4 carbon atoms.

2. Methanonaphthalenes according to claim 1, characterised in that R signifies methyl or ethyl.

3. Process for the production of methanonaphthalenes of the formula

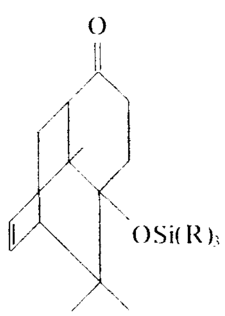

(V)

characterised in that a compound of the formula

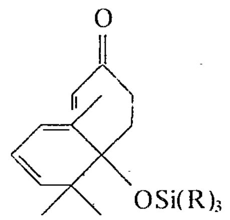

(IV)

wherein R signifies a lower alkyl group, phenyl optionally lower alkyl-substituted or phenyl-lower alkyl, wherein the lower alkyl groups contain 1—4 carbon atoms

is intramolecularly cyclised to give a compound of the formula V.

4. Process according to claim 3, characterised in that a starting material is used wherein R signifies a methyl or ethyl group.

5. Process according to claim 3 or 4, characterised in that the cyclisation is carried out in the liquid phase.

6. Process according to claim 5, characterised in that the cyclisation is performed in an inert organic solvent.

7. Process according to claim 6, characterised in that an aromatic or aliphatic hydrocarbon is used as the organic solvent.

8. Process according to one of claims 3—7, characterised in that the cyclisation is carried out at a temperature between 200 and 300° C.

9. Process according to claim 8, characterised in that the cyclisation is carried out at a temperature between 220 and 250° C.

10. Process according to claim 3 or 4, characterised in that the cyclisation is carried out in the gas phase.

11. Process according to claim 10, characterised in that the cyclisation is carried out at a temperature between 500 and 800° C.

12. The use of methanonaphthalenes of the formula

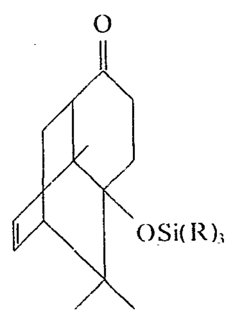

(V)

wherein R signifies a lower alkyl group, phenyl optionally lower alkyl-substituted or phenyl-lower alkyl, wherein the lower alkyl groups contain 1—4 carbon atoms

for the preparation of norpatchoulenol of the formula

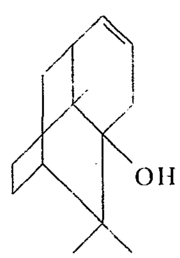

(VIII)

characterised in that, in any desired order, the trisubstituted silyl group is cleaved off, the 7,8-double bond is hydrogenated to a single bond, a hydrazone derivative of the thus-formed 4-keto compound is formed and the hydrazone group is removed with formation of a 3,4-double bond, whereby norpatchoulenol is obtained.

13. Use according to claim 12, characterised in that the trisubstituted silyl group is cleaved off by mild acid hydrolysis.

14. Use according to claim 12 or 13, characterised in that the hydrogenation of the 7,8-double bond is carried out with the use of a noble metal catalyst.

15. Use according to claim 14, characterised in that a platinum or palladium catalyst is used as the noble metal catalyst.

16. Use according to one of claims 12—15, characterised in that a p-toluenesulphonylhydrazone is formed as the hydrazone derivative.

17. Process according to one of claims 12—16, characterised in that the starting material of the formula IV in the S-configuration is used, whereby (+)-norpatchoulenol is obtained as the end product of the formula VIII.

**Revendications**

1. Méthanonaphtalines de formule

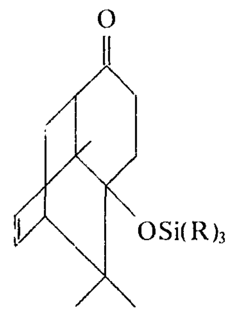

(V)

où R représente un groupe alcoyle inférieur, un phényle éventuellement alcoyle inférieur-substitué ou un phénylalcoyle inférieur, où les groupes alcoyle inférieurs contiennent de 1 à 4 atomes de carbone.

2. Méthanonaphtalines selon la revendication 1, caractérisées en ce que R représente un méthyle ou un éthyle.

3. Procédé de préparation de méthanonaphtalines de formule

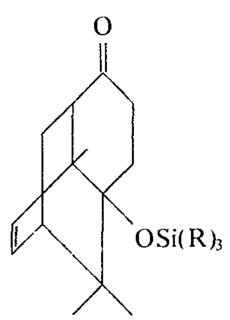

(V)

caractérisé en ce qu'on cyclise un composé de formule

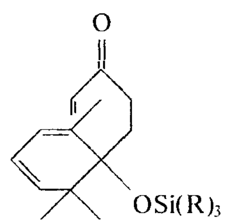

(IV)

où R représente un groupe alcoyle inférieur, éventuellement un phényle alcoyle inférieur-substitué ou un phényl-alcoyle inférieur, où les groupes alcoyle inférieur contiennent de 1 à 4 atomes de carbone,

et ce de façon intra-moléculaire pour donner un composé de formule V.

4. Procédé selon la revendication 3, caractérisé en ce qu'on utilise un produit de départ où R représente un groupe méthyle ou éthyle.

5. Procédé selon les revendications 3 ou 4, caractérisé en ce qu'on effectue la cyclisation en phase liquide.

6. Procédé selon la revendication 5, caractérisé en ce qu'on effectue la cyclisation dans un solvant organique inerte.

7. Procédé selon la revendication 6, caractérisé en ce qu'on utilise comme solvant organique un hydrocarbure aromatique ou aliphatique.

8. Procédé selon l'une des revendications 3 à 7, caractérisé en ce qu'on effectue la cyclisation à une température comprise entre 200 et 300° C.

9. Procédé selon la revendication 8, caractérisé en ce qu'on effectue la cyclisation à une température comprise entre 220 et 250° C.

10. Procédé selon les revendications 3 ou 4, caractérisé en ce qu'on effectue la cyclisation en phase gazeuse.

11. Procédé selon la revendication 10, caractérisé en ce qu'on effectue la cyclisation à une température comprise entre 500 et 800° C.

12. Application de méthanonaphtalines de formule

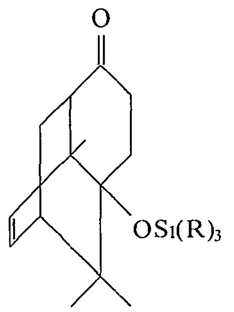

(V)

où R représente un groupe alcoyle inférieur, un phényle éventuellement alcoyle inférieur-substitué ou un phénylalcoyle inférieur, où les groupes alcoyle inférieurs contiennent de 1 à 4 atomes de carbone

à la préparation du norpatchoulénol de formule

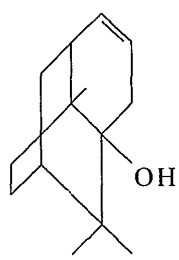

(VIII)

caractérisée en ce que, dans un ordre quelconque, on sépare le groupe silyle tri-substitué, on hydrogène la double liaison 7, 8 en une liaison simple, on forme un dérivé d'hydrazone du composé 4-céto ainsi formé et on dégrade le groupe hydrazone avec formation d'une double liaison 3,4 ce qui donne le norpatchoulénol.

13. Application selon la revendication 12, caractérisée en ce qu'on sépare le groupe silyle trisubstitué par hydrolyse faiblement acide.

14. Application selon la revendication 12 ou 13, caractérisée en ce qu'on effectue l'hydrogénation de la double liaison 7,8 en utilisant un catalyseur en métal noble.

15. Application selon la revendication 14, caractérisée en ce qu'on utilise comme catalyseur en métal noble un caralyseur au platine ou au palladium.

16. Application selon l'une des revendications 12 à 15, caractérisée en ce qu'on forme comme dérivé d'hydrazone une p-toluène-sulfonylhydrazone.

17. Application selon l'une des revendications 12 à 16, caractérisée en ce qu'on utilise le produit de depart de formule IV en configuration S, et en ce qu'on obtient comme produit final de formule VIII le (+)-norpatchoulénol.